# EUROPEAN PATENT SPECIFICATION

(11) **EP 2 210 567 B1**
(45) Date of publication and mention of the grant of the patent: **31.07.2013**
(21) Application number: 09151319.2
(22) Date of filing: 26.01.2009
(51) Int. Cl.: A61B 18/12, A61B 18/14

(54) **A bipolar electrosurgical instrument**
Bipolares elektrochirurgisches Instrument
Instrument électro-chirurgical bipolaire

(43) Date of publication of application: 28.07.2010
(73) Proprietor: Lina Medical ApS, 2600 Glostrup (DK)
(72) Inventor: Kornerup, Niels, 5871, Frørup (DK)
(74) Representative: Hertling, Peter

(56) References cited:
- WO-A-2007/005830
- WO-A-2008/010150
- WO-A-2008/063195
- GB-A- 2 374 532
- US-A1- 2003 233 088

## Description

The present invention relates to a bipolar electrosurgical instrument being connectable to an electrosurgical generator, said bipolar electrosurgical instrument comprising a hollow shaft sized to fit through a laparascopic port and having a proximal end and a distal end, a loop electrode constituted by an electrically conductive resilient looped wire provided with a first means for reciprocating the loop electrode between a retracted position inside the hollow shaft and a second position in which at least a part of the loop electrode is emerged from the distal end of the hollow shaft, and at least two return electrodes provided with second means for reciprocating the at least two return electrodes between a retracted position inside the hollow shaft and a second position in which at least a part of the return electrodes are emerged from the distal end of the hollow shaft wherein, in use,
(a) the looped wire of the loop electrode can be arranged around a tissue object when the loop electrode is in the second position,
(b) and can then be constricted by retracting the loop electrode towards the retracted position, whereby the return electrodes obtain direct contact with the tissue object

More particularly the invention relates to a bipolar electrosurgical instrument for laparoscopic hysterectomy.

Performing a laparoscopic hysterectomy requires the use of electrosurgical instruments, a.o. to cut, coagulate, desiccate or fulgurate tissue. A particular need is to make precise cuts with limited blood loss. To that aspect the conventional electrosurgical instruments are either monopolar or bipolar. Both kinds of electrodes involve high frequency alternating current and a pair of electrodes, in the following referred to as an active electrode and a return electrode. The difference in the two principles lies in the placement of these electrodes.

Monopolar techniques rely on external grounding of the patient, where the surgical device defines only a single electrode pole. Bipolar devices have two electrodes for the application of current between their surfaces.

In monopolar electrosurgery the patient lies on top of the return electrode, e.g. a large metal plate, or a smaller return electrode plate is attached to the tight of the patient. The return electrode is maintained at ground potential. The surgeon uses an active electrode to contact the tissue in order to cut and coagulate the tissue. The electrical current flows from the tip of the active electrode through the body and then to the return electrode, from which it flows back to the electrosurgical generator. Heating is confined to the tissue that is near the tip of the active electrode beacuse the current rapidly spreads out laterally as it enters the body, causing a dramatic decrease in the current density. Monopolar electrosurgery relies on a good electrical contact between a large area of the patient's body and careful application of the return electrode is necessary. The current may inadvertently flow along body paths having less impedance than the defined electrical path, which will substantially increase the current flowing through these paths, possibly causing damage to the patient. Thus if the monopolar electrosurgery is not correctly performed extensive third degree burns can occur in unintended areas on the patients skin and beneath the skin in vital organs and there the risk of capacitive coupling is higher than for bipolar electrosurgery. Monopolar electrosurgery is therefore unsuited for several internal electrosurgical cutting actions.

A bipolar electrosurgical instrument as the one disclosed in the applicant's own international patent application no. PCT/IB2007/052735 is, in contrast to monopolar electrosurgical instruments, suitable for performing repeated internal cutting actions.

In bipolar electrosurgery the active electrode and return electrode are both placed at the surgical site. For example the two opposing jaws of a forceps or the two legs of a tweezer serve as the two opposing electrodes. Voltage is applied to the patient with the active electrode connected to the positive side of the voltage source and the return electrode connected to the negative side of the voltage source. When a piece of tissue is located between the electrodes an electrical current flows from the positive electrode to the negative electrode through the tissue and the tissue is heated and cutting effectuated paying attention not to unintentionally cause damage to healthy tissue in close proximity to the object. Thus, in bipolar electrosurgical devices, both the active electrode and return electrode are typically exposed so that both electrodes are able to contact tissue, thereby providing a controlled return current path from the active to the return electrode through the tissue.

However, still there remains a need within the art to improve electrosurgical techniques towards eliminating unintended effects and disadvantages related to their use.

For further discussion of the monopolar and bipolar electrosurgical techniques and medical conditions reference is e.g. made to the applicant's international patent application PCT/IB2007/052735, published as WO 2008/010150 A2, also relating to an electrosurgical instrument useable for performing laparoscopic hysterectomies. There are a variety of surgical techniques for performing hysterectomies including total hysterectomy, vaginal hysterectomy, laparoscopy-assisted vaginal hysterectomy (LAVH), supracervical, laparoscopic supracervical hysterectomy (LASH), and radical hysterectomy. The type of hysterectomy performed is dependant upon the woman and the reason for the procedure and with a view to reduce complications and side effects. Other examples of laparoscopic procedures include removal of larger tissue in myomectomy, oophorectomy and salpingo-oophorectomy.

One advantage of laparoscopic procedures is that the incisions are small and much less uncomfortable than that of open surgery, however an average uterus size is about 8 cm long, 5cm wide and 2.5 cm thick and even larger in a woman who has had a child, and cannot be removed in one piece through e.g. a 10 mm diameter trocar. Several severing actions or degrees of comminution are therefore needed if the object shall be removed one piece after another or as a "soup".

The most preferred technique is to use a morcellator, e.g. electrosurgical morcellators especially designed to cut the uterus free from the cervix.

US patent application no. 5,520,634 discloses an example of a mechanical morcellator, which includes a rotatable and relatively retractable cutting head. User manipulation varies the amount the cutting head extends out of a sheath, the amount of suction communicated to the cutting head, and the operation of a motor which rotatably drives the cutting head. Suction is communicated to the cutting head to aspirate the tissue fragmented by the cutting head. The morcellator is adapted to be inserted through a laparoscopic port site and directly fragment and aspirate tissue from within a patient's body. This mechanical morcellation technique is not very popular because the pathologist cannot use the morcellated tissue for the majority of pathological purposes. Moreover, the technique will prolong the surgical operation with approximately 15 min. Finally, the mechanical morcellator can be dangerous in non-skilled hands. During morcellation cells which are potential infectious or malign are susceptible to be spread inside the abdominal cavity. This cannot be prevented, even though attempts are made to confine the morcellated tissue in a receptacle inserted through a separate trocar. Furthermore, it is difficult if not impossible to identify the nature of the malignancy and the location of the malignancy on the uterus if the tissue is morcellated. As a result the surgeon, pathologist and the patient are deprived many of the information's which could be retrieved from analysing an intact object, information which is useful in the treatment regimen for reducing the risk of metastasis and infections and for providing prophylactic treatment.

These adverse effects are highly undesired and there exists a need for a safe and reliable electrosurgical instrument for performing several bipolar electrosurgical cutting actions inside the body, for example when performing laparoscopic hysterectomies, in particular for performing laparoscopic supracervical hysterectomy (LASH) where a fundusectomy of the uterus is made, leaving the cervix in place.

In a main aspect according to the present invention is provided a surgical instrument of the kind mentioned in the opening paragraph that is safer to use than known electrosurgical instruments.

It is a second aspect of the present invention to provide a surgical instrument of the kind mentioned in the opening paragraph that can be used for several consecutive electrosurgical internal bipolar electrosurgical cuttings during the same electrosurgical procedure.

In a third aspect of the present invention is provided an electrosurgical instrument of the kind mentioned in the opening paragraph, which applies current to the active electrode for a reduced period of time during an electrosurgical procedure.

In a fourth aspect of the present invention is provided an electrosurgical instrument of the kind mentioned in the opening paragraph in which an active electrically conductive loop electrode can be forwarded to and retracted from the electrosurgical site a number of times to electrosurgically cut an object having a cross-section larger than the cross-section of a laparoscopic port into smaller parts with a minimum or none damage to the surrounding body.

In a fifth aspect of the present invention is provided a surgical instrument of the kind mentioned in the opening paragraph that can be operated solely by the hands and is more user friendly than known devices.

The novel and unique whereby this is achieved according to the present invention is the fact that the bipolar electrosurgical instrument further comprises a third means for measuring the impedance, resistance or capacity in an object situated between the at least two return electrodes when the return electrodes are in direct contact with the tissue object, and a fourth means for applying current to the loop electrode when said value of the impedance, resistance or capacity is below a predetermined value and/or in a predetermined period of time.

The loop electrode is the active electrode used for bipolar electrosurgical cutting of an object such as the uterus. The emerged loop electrode and the emerged return electrodes are in close proximity but not in direct contact with each other. In order to perform a safe bipolar electrosurgical cutting action the loop of the loop electrode is arranged around the object and the loop is constricted by retracting the loop electrode towards the proximal end of the hollow shaft. No current is applied to the loop of the loop electrode at this stage. As a result of retracting the loop of the loop electrode the return electrodes obtains direct contact with the object. In this position the third means is actuated to measure or registers automatically the value of the impedance, resistance or capacity or similar value between the return electrodes, which value is indicative of the presence of an object in the loop. If the value is below a predetermined limit, which e.g may be in the interval of 5 - 150.000 Ω, the third means provides a signal that bipolar electrosurgical cutting can be perfected. The fourth means is now activated, e.g. by surgeon, thereby providing a current flow through the loop of the now active loop electrode, applying a secure voltage between the loop electrode and the return electrode. As a result heat is applied at the bipolar electrosurgical cutting site. While current is flowing in the loop of the loop electrode, the final bipolar electrosurgical cutting is perfected by further retracting the active, electrically loop back into the hollow shaft beyond the return electrodes. Once the tissue, e.g. the uterus is cut free, the return electrodes is out of contact with the tissue object and the value of the impedance, resistance or capacity again exceeds the selected predetermined value and the return electrodes are ready for new use. This increase is recorded and can be read on e.g. a display on a electrosurgical generator or can be directly read on a display on the electrosurgical instrument itself. Accordingly, the value of the impedance, resistance or capacity is used for either automatically or manually disconnect or connect power supply to the loop electrode. Thus according to the present invention power is applied to the loop electrode for a much shorter period than when using conventional bipolar electrosurgical instruments.

The fourth means is preferably an electrosurgical generator including one or more high frequency power sources and an output stage including output lines for connection to the electrosurgical instrument according to the present invention and preferably various display for monitoring applied voltage and any of the value of the impedance, resistance or capacity as the case may be.

The generator may be of the kind including a controller that controls the generator to deliver a suitable high frequency waveform for use as a bipolar electrosurgical cutting signal to the output lines. An exemplary suitable electrosurgical generator delivers as an example a power of 100 W at an output frequency of 500 kHz and applying a voltage of 6.000 V.

When the return electrodes are arranged coaxilly to the loop electrode, at least when the loop electrode is emerged from the proximal end of the hollow shaft, the object can be situated with substantially the same distance to the return electrodes once the active loop electrode is arranged around the loop, to further ensure that no unintended current flow paths are achieved when current flows through the loop during bipolar electrosurgical cutting.

At least a part of the loop of the loop electrode may be arranged to be emerged between the emerged at least one return electrodes leaving a part of the loop inside the shaft. The thereby obtained flexibility and inherent spring-like capability of the loop makes the loop to spring open to unfold and maintain between the return electrodes when the loop is emerged from the distal end of the hollow shaft and to be retracted again without getting entangled with the return electrodes. At least the emergeable part of the loop of the loop electrode made be shaped as a rhombus. The emergeable loop electrode may for example be made of a stainless steel wire having an uninsulated substantially V-shaped or U-shaped cutting part extending into an insulated part situated between the at least one return electrodes. The length of the cutting part of the loop of the loop electrode may typically be about 40 mm but this example of a length is not exhaustive and may vary within the scope of the present invention as well as the length of the entire emergeable loop may vary and be selected according to specific uses. It is evident to the skilled surgeon that if a very large object is to be removed and sectioned inside the body a longer loop is required than if only a small object needs to be removed and sectioned.

The bipolar electrosurgical instrument may in the simple embodiment comprise two return electrodes, but in a preferred embodiment the bipolar electrosurgical instrument comprises at least four return electrodes, three of which is electrically interconnected to constitute a single return electrode having a larger contact surface to the object. So by providing four return electrodes around the object good contact with the object is achieved and a reliable measurement of the object's impedance, resistance or capacity can be ensured to promptly inform the surgeon that safe bipolar electrosurgical cutting may take place. An electrosurgical instrument provided with more than four return electrodes may be preferred, e.g if the object to be cut is very irregular. In such cases the return electrodes are divided in two sets of several return electrodes which are electrically interconnected, to provide a system of two active return electrode parts between which the value of the impedance, resistance or capacity can be established.

In the most preferred embodiment of the inventive electrosurgical instrument the return electrodes can be constituted by flat springy, divergingly arranged conductive plates or blades, preferably of stainless steel, so that the return electrodes opens as a flower bursting when the return electrodes are emerged from the distal end of the hollow shaft and so that the return electrodes easily can be retracted inside the hollow shaft using a minimum of pulling force. The "flower arrangement" of the return electrodes provides a good resting surface for the object when measuring of the value of the impedance, resistance or capacity in the object takes place.

When an object, for example the uterus, is situated inside the active loop electrode and between the return electrodes the resistance, impedance or the capacity between the return electrodes decreases, and the third means may register that the predetermined value of the resistance or impedance is for example in the interval between 5 - 150.000 Ω which is an indication that bipolar electrosurgical cutting can be made and that current can be applied to the active loop electrode to establish a voltage consistent with a quick bipolar electrosurgical cutting action. As soon as bipolar electrosurgical cutting is performed the surgeon is able to confirm his visual observation of finalised bipolar electrosurgical cutting by retrieving information of the value of the impedance, resistance or capacity. If this value is higher than the predetermined value this indicates perfected bipolar electrosurgical cutting. The surgeon may even dispense with visual verification of finalising the bipolar electrosurgical cutting action. The configuration and arrangement of the return electrodes provides a greatly demanded additional safety mechanism ensuring that current is only floating through the loop electrode during the actual bipolar electrosurgical cutting process. The risk of injury is self-evident in that current is flowing in a much shorter interval in a surgical procedure in which the surgeon has more control of application of voltage than when using known devices. The arrangement allows the surgeon to perform several consecutive, bipolar, cuttings inside the body.

The fourth means for applying power to the loop electrode and the third means for measuring any of the impedance, the resistance or capacity may preferably be integrated in an electrosurgical generator so that only one unit besides the bipolar electrosurgical instrument needs to be worked during surgery. The electrosurgical generator may be provided with instrumentations such as knobs, displays etc, to provide a clear indication of which part of the generator that must be operated and switched on and off during surgery, and preferably the electrosurgical instrument is configured so that most of the manipulations of the electrosurgical generator which are required for the electrosurgery can be actuated from the bipolar electrosurgical instrument, so that the surgeon can keep his eyes on the surgical site during the laparoscopic procedure.

To improve operating the bipolar electrosurgical instrument the hollow shaft may advantageous comprise an outer tubular casing having a longitudinal slide groove for reciprocating the return electrodes, and an inner tubular casing accommodating the loop electrode which inner tubular casing is insulated from the outer tubular casing. The length of the slide group is adjusted and adapted to allow for a suitable but restricted pushing forward and retracting of the system of return electrodes and should be arranged in the wall of the outer tubular casing so that the surgeon has unobstructed access to a grip means for manipulating moving of the return electrodes. If the object to be cut off is rather small the surgeon has the option of limiting the forward movement of the return electrode to thereby keep the distance between each return electrode smaller than in the fully emerged condition. Thus for small objects the surgeon may chose not to spread the return electrodes completely apart and keep a smaller distance between them.

This disclosure further relates to a novel method of performing laparoscopic detachment of an object from its attachment.

The method comprises the steps of
a. introducing the electrosurgical instrument as described above through a laparoscopic port site,
b. emerging the at least two return electrodes from the distal end of the hollow shaft,
c. emerging the loop electrode from the distal end of the hollow shaft to create a loop for surrounding at least a part of an object,
d. tightening the loop around at least a part of an object by pulling the loop electrode towards the proximal end of the hollow shaft to set the object in contact with the at least two return electrodes,
e. measuring the impedance, resistance or capacity of the object between the at least two return electrodes,
f. if the impedance, resistance or capacity is below a predetermined value applying power to the loop electrode, and
g. maintaining the power supply to the loop electrode while performing a bipolar electrosurgical cutting action by further retracting the loop electrode towards the proximal end of the hollow shaft.

The steps of the disclosed method will be thoroughly discussed during detailed description of the accompanying drawing.

In order to complete the bipolar electrosurgical cutting action and ensure that the object is cut loose and/or sectioned appropriately step g may preferably include that the loop electrode is retracted inside the hollow shaft until the loop electrode is out of contact with the object before the power supply is switched off.

If the object that is cut off is to be removed via a laparoscopic port, a trocar, the object needs to be divided in smaller parts. To this aspect the method may advantageously comprise repeating at least steps b - g until the object is divided into a plurality of minor pieces having a cross-section allowing passage through a laparoscopic port, such as a trocar. The smaller pieces may be colleted in a receptacle to subsequently be removed via the incision for the trocar after the trocar has been removed.

Test has revealed that a if the number of return electrodes are at least four a good contact surface with the object to be cut can be obtained and maintained during bipolar electrosurgical cutting, however less than four may be suitable to some purposes.

The method according to the present disclosure is particular suited for laparoscopic detachment procedures, such as hysterectomies, in particular a laparoscopy-assisted vaginal hysterectomy which is made through a laparoscopic port and therefore needs several consecutive bipolar electrosurgical cutting actions. Typical values are 6.000 V, 100W at 500 kHz.

The invention will be described in further details below with reference to the accompanying drawing illustrating an exemplary embodiment of the bipolar electrosurgical instrument according to the invention in various steps of the bipolar electrosurgical method. In the embodiment shown in the figures the bipolar electrosurgical instrument has four conductive return electrode blades, however within the scope of the present invention any suitable number equal to or more than two can be used.

The shaft of the bipolar electrosurgical instrument is shown to be partly transparent only for illustrative purposes, but can have any degree of transparency including none.
Fig. 1 shows a general view, seen in perspective, of a surgical generator and a bipolar electrosurgical instrument according to the present invention and in a situation where both the loop of the loop electrode and the return electrodes are emerged from the distal end of the hollow shaft corresponding to having performed method steps b and c,
Fig. 1a shows, seen in perspective and in enlarged scale, the proximal end of the bipolar electrosurgical instrument shown in fig. 1,
Fig. 1b shows, seen in perspective and in enlarged scale, the distal end of the bipolar electrosurgical instrument shown in fig. 1,
Fig. 2 shows, seen in perspective and in a fragmentary view, the bipolar electrosurgical instrument in a situation ready to use where both the loop of the loop electrode and the return electrodes are retracted inside the hollow shaft,
Fig. 3 shows, seen in perspective and in a fragmentary view, the bipolar electrosurgical instrument with the return electrodes emerged, corresponding to step b,
Fig. 4 corresponds substantially to fig. 1, but in an enlarged fragmentary view to better improve understanding,
Fig. 4a shows, in an even more enlarged scale, another detail of the distal end of the hollow shaft, of the method step c shown in fig. 4,
Fig. 5 shows, seen in a perspective fragmentary view, how the loop of the loop electrode is arranged to encircle the object to be cut, ready to perform step d,
Fig. 6 shows in a perspective fragmentary view how the loop of the loop electrode is tightened around the object to be cut while performing step d, e and f,
Fig. 7 shows, seen in perspective, step g where the object has been cut in two pieces and the loop electrode is fully retracted inside the hollow shaft again.

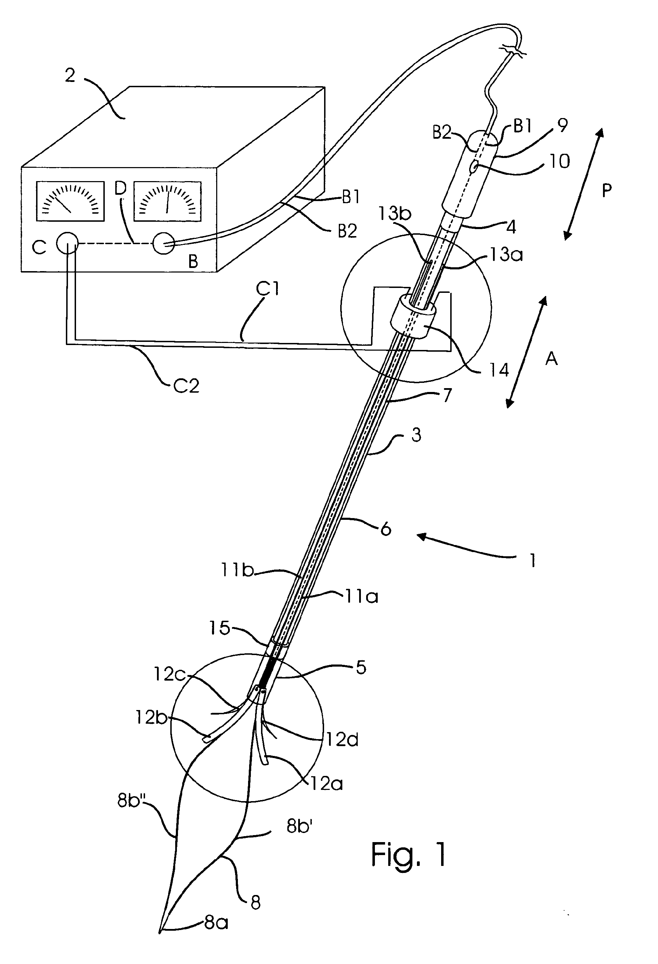

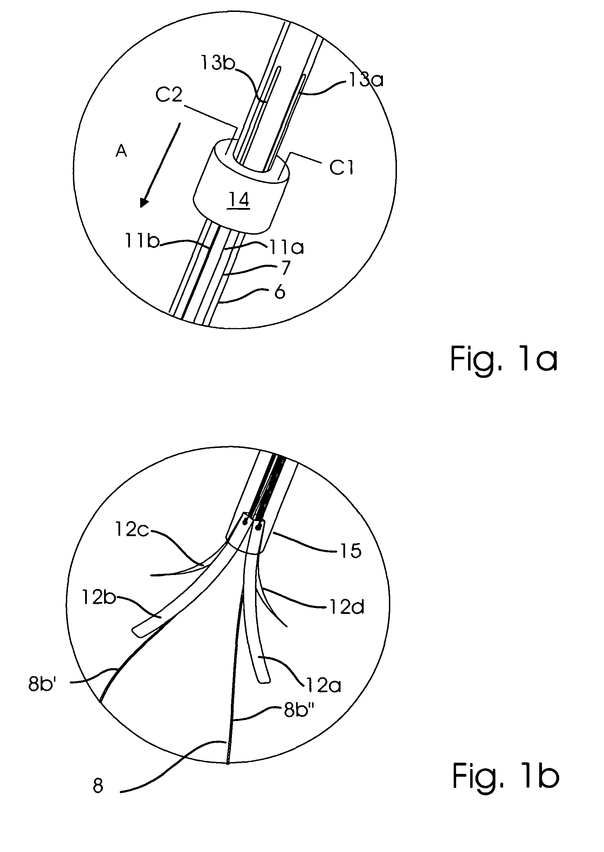

Fig. 1 shows, in perspective, a general view of a bipolar electrosurgical instrument 1 connected to an electrosurgical generator 2. The bipolar electrosurgical instrument 1 has a hollow shaft 3 with a proximal end 4 and a distal end 5. The hollow shaft 3 is defined at least by an outer tubular casing 6 inside which an inner tubular casing 7 extends reciprocatingly. Typically, the outer tubular 6 casing has an exterior diameter of about 10 mm to ensure that it can pass smoothly through a conventional laparoscopic trocar port of same diameter. The inner tubular casing 7 accommodates, as indicated by dashed line, the loop electrode 8, such as an electrically conductive resilient looped wire, which is electrically connected to the electrosurgical generator 2, exemplified at circuit B, via electric wire B1, to enable electrosurgical cutting. A first handle 9 at the proximal end of the inner tubular casing 7 has an actuation knob 10 which is connected to circuit B via electric wire B2 to enable the surgeon to switch cutting current on and off on demand and according to the surgeons choice only by a simple pressure on the actuation knob 10. The first handle is used for reciprocating the loop electrode as indicated with arrow P in the figures.

The loop of the loop electrode 8 has an uninsulated cutting part 8a of e.g. 40 mm and an insulated part 8b ensuring that heat application to an object is confined to a limited and controlled area still maintaining a firm grip at the object, e.g. the uterus.

Circuit B may be implemented in a separate device to be used together with a conventional bipolar electrosurgical generator and the electrosurgical instrument according to the present invention, or circuit B can be integrated in a new electrosurgical generator specifically designed for the present invention.

Four return electrodes 11a,11b,11c,11b, of which only two are visual in fig. 1, extends slidingly lengthwise in the circumferential space between the inner tubular casing 7 and the exterior tubular casing 6 in recesses in one or more spacer plugs 15, of which only one can be seen in fig. 1. Thus the return electrodes 11a,11b,11c,11b are isolated from the loop electrode 8. Each return electrode 11a,11b,11c,11d have a distal end configured as a flat conductive plate or blade 12a,12b,12c,12d of enlarged surface area to establish good contact with an object (not shown) to be cut. The proximal end of three of the return electrodes e.g. 12a,12b,12c are mutually connected to obtain one common reference electrode 12a,12b,12c which, via electric wire C2, is coupled to a circuit C arranged in the electrosurgical generator 2. The last of the four return electrodes 12d is coupled to circuit A via electric wire C1, thus when an object (not shown) is situated between the spread apart object contacting return electrodes it is possible to establish a value of the impedance, resistance or capacity to be used as an indication of the presence of an object to inform the surgeon that cutting current can be switched on, or off if no contact is established. If the value is above a predetermined value it indicates that no object is located inside the loop vice versa. Any combination of return electrodes are foreseen within the scope of the present invention as long as the bipolar electrosurgical instrument has two electrically conducting set of return electrodes.

Transmission of a signal representing the measured impedance, resistance or capacity from circuit C to circuit B is in the case shown integrated in the bipolar electrosurgical generator and takes place via dashed line D.

As shown in an enlarged scale in fig. 1a the outer tubular 6 casing has at its proximal end 4 a number of longitudinal slide grooves 13a,13b,13c,13d, og which only two can be seen in fig. 1 and fig. 1a. The number of slide grooves 13a,13b,13c,13d corresponds to the number of return electrodes 11a,11b,11c,11d. The proximal ends of the return electrodes 11a,11b,11c,11d are passed through the corresponding slide grooves 13a,13b,13c,13d and secured to a second handle 14 for reciprocating, as indicated with the arrow A, said return electrodes 12a,12b,12c,12d in and out of the distal end 5 of the hollow shaft 3 as occasion requires.

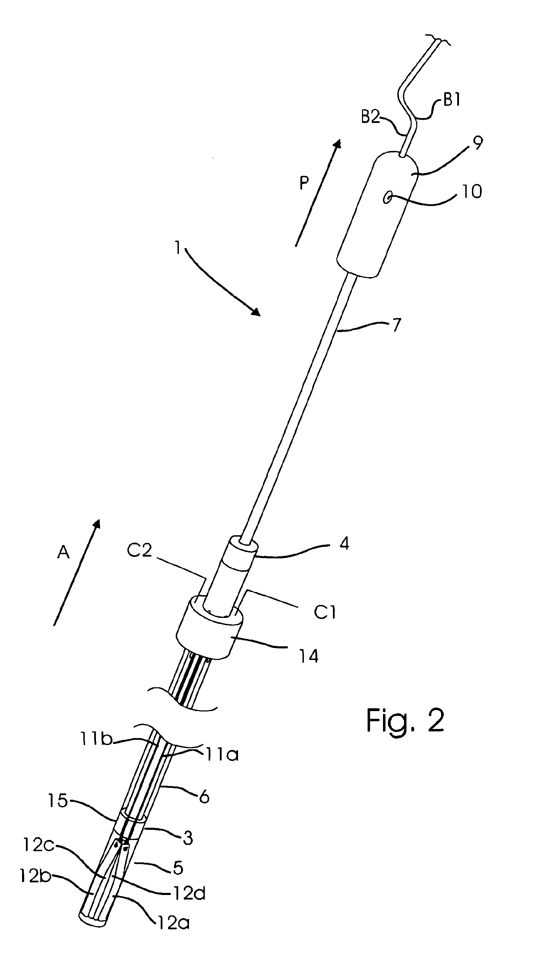

As seen better in the enlarged scale view of fig. 1b the flat conductive blades or plates 12a,12,b,12c,12d of the return electrodes 11a,11b,11c,11d are curving outwards when emerged from the distal end 5 of the hollow shaft 2 and surrounding the loop electrode 8. The curvature should be sufficient to provide the flat conductive blades or plates 12a,12b,12c,12d with sufficient spring force to by themselves and instantly spread apart as a flower opens when emerged from the distal end 5 of the hollow shaft 3 and sufficiently flexible to yield to be retracted inside said distal end again as shown in the fragmentary perspective view of fig. 2. Although the conductive blades of the return electrodes are shown as flat rectangular plates other outlines and geometries are foreseen within the scope of the present invention.

For clarity reasons the subsequent figures do not indicate wiring and electrosurgical generator but serve for better illustrating the method according. Due to the enlarged scale these figures are fragmentary.

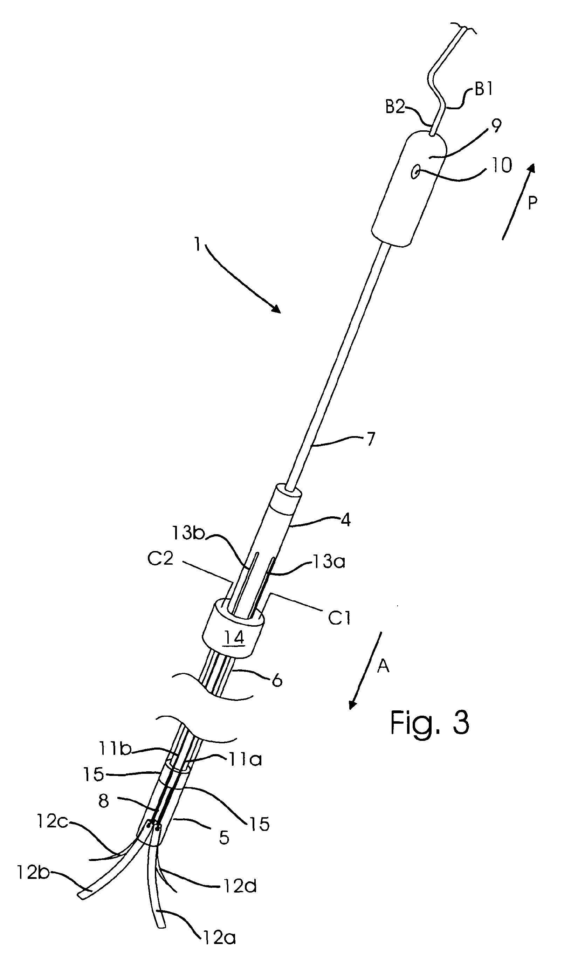

Fig. 3 shows, seen in perspective and in a fragmentary enlarged view, the bipolar electrosurgical instrument 1 with the return electrodes 11a,11b,11c,11d emerged and the loop electrode 8 retracted, corresponding to step b of the method according to the disclosure. The second handle 14 is manually advanced towards the distal end 5 of the hollow shaft 3 so that the distance to the first handle 9 is increased but still leaving the loop electrode 8 hidden inside the distal end 5.

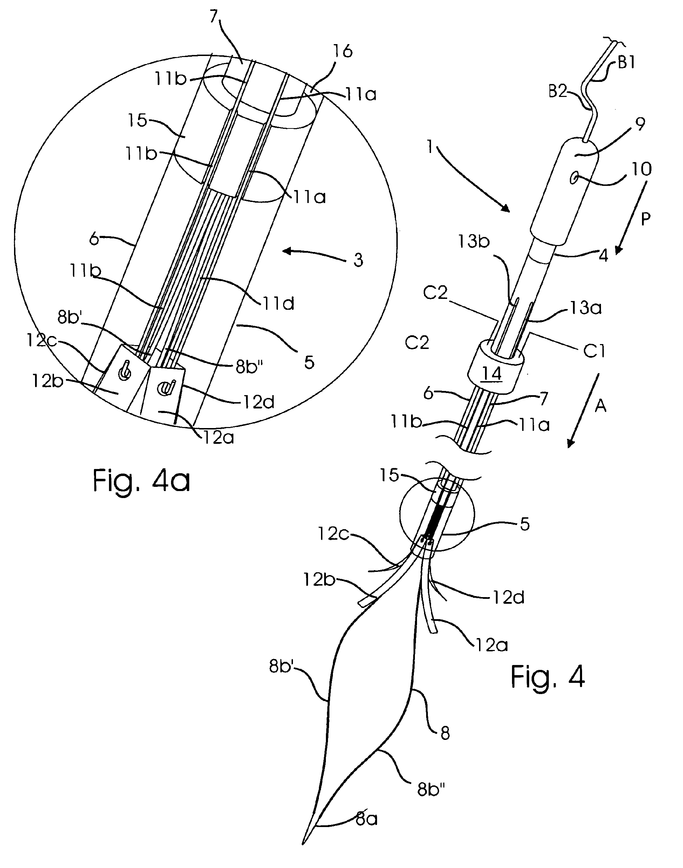

The further step c of emerging the loop electrode 8 from the distal end of the hollow shaft is seen better in the enlarged scale fragmentary view of fig. 4, which corresponds substantially to fig. 1, but is presented to better overview the many details of the instrument.

Fig. 4a shows the method step c shown in fig. 4, in an even more enlarged scale how the two legs 8b',8b " of the loop electrode 8 extends lengthwise inside the outer tubular casing 6 between the return electrodes 11a,11b,11c,11d. The return electrodes 11a,11b,11c,11d are firmly secured to the flat conductive blades 12a,12b,12c,12d. In the exemplary embodiment shown in the figures the return electrodes 11a,11b,11c,11d are long conductive rods securely hooked to flat conductive plates 12a,12b,12c,12d, however the return electrode may quite as well be a manufactured as a single unit or the rods may be welded to the plates or any other suitable conductive coupling together. Fig. 4a also illustrates how the guide plug 15 at the distal terminal end of the inner tubular casing 7 serves for holding the return electrodes 11a,11b,11c,11d in the circumferential space 16 between the outer tubular casing 6 and the inner tubular casing 7.

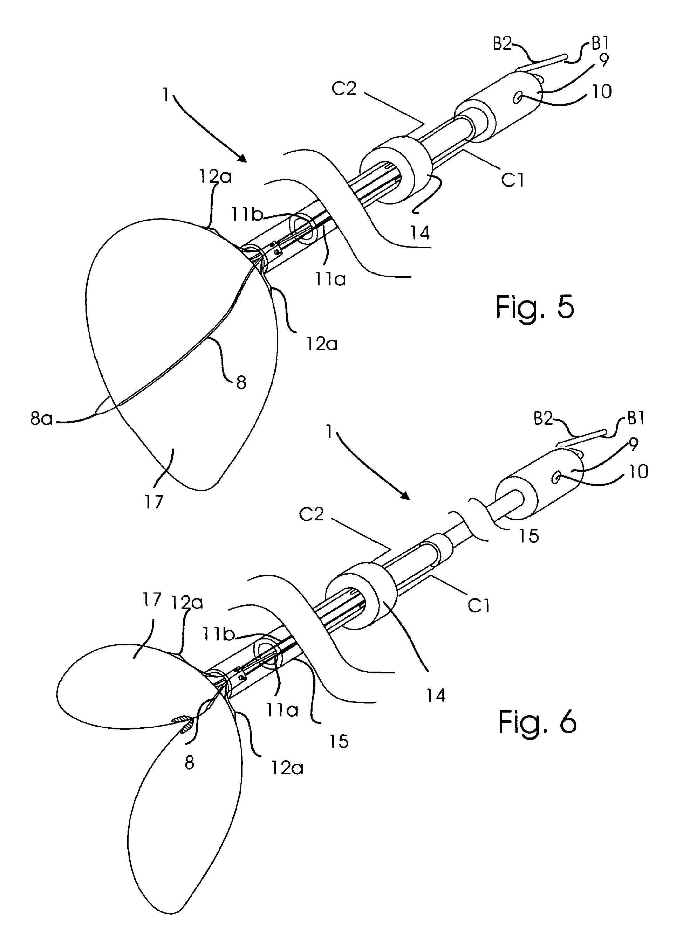

Figs. 5, 6 and 7 shows in a perspective fragmentary view the intermediate situations showing step c where the loop of the loop electrode is arranged to encircle an exemplary indicated object 17 to be cut to step g where the object has been divided in tow parts.

Fig. 5 shows the touching location of the object 17 between the return electrodes 11a,11b,11c,11d decreasing the value of the impedance, resistance or capacity measured by circuit C of the bipolar electrosurgical generator 2. Once circuit C has measured the size of the value of the impedance, resistance or capacity it compares the measured value with a predetermined value. If the measured value is below or equal to the predetermined value circuit C provides a signal to the surgeon, such as e.g. by means of a signal lamp on the electrosurgical generator flashing green, indicating that step d - g can be initiated to start the bipolar electrosurgical cutting procedure.

The tightening of the loop electrode 8 around the object 17 according to method step d is illustrated in fig. 6. By actuating the knob 10 on the first handle 9 to switch on power to the loop electrode the surgeon can now proceed with the electrosurgical cutting assured by means of the signal provided by circuit C that steps e-g can be safely made. Continous measuring of the impedance, resistance or capacity value is performed during the bipolar electrosurgical cutting either until the value is below or equal to the predetermined value or for a defined period of time to thereby provide the signal to stop cutting, e.g. triggered manually by the surgeon releasing the actuation knob 10 in response to an alert from the bipolar electrosurgical generator 2, or automatically in response to the same. Thus current is thus applied to the loop electrode 8 only a very short period in which the return electrodes acts both as a safety mechanism and a conventional bipolar return electrode.

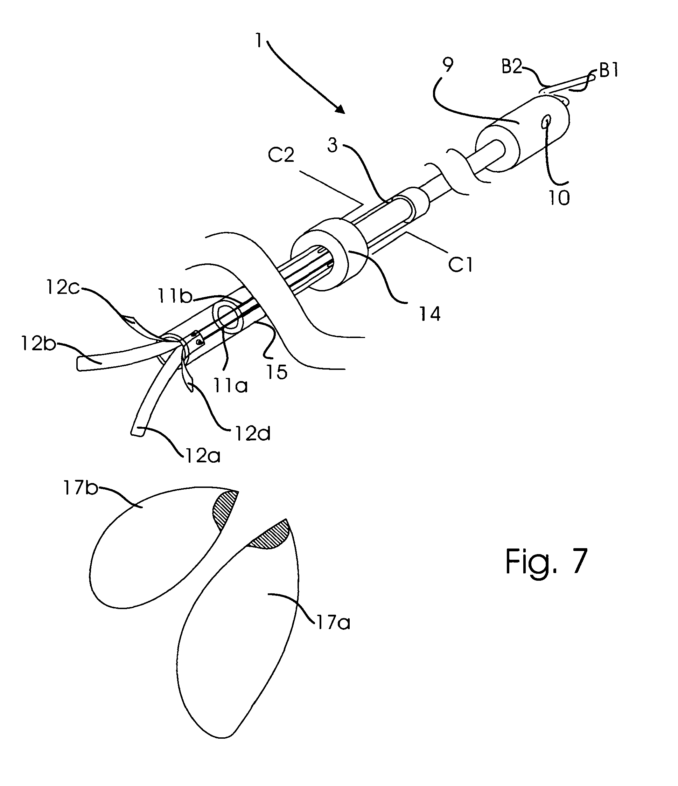

Fig. 7 shows, seen in perspective, step g, where the object 17 has been cut in two pieces 17a,17b and the loop electrode 8 is fully retracted inside the hollow shaft 3 again. The pieces 17a,17b are catched and hold by a forceps introduced through another laparoscopic port. The forceps has a firm grip at the object 17 or pieces 17a,17b of object so that the method can be repeated for as many bipolar electrosurgical cuttings as required, e.g. until the object is cut into 10-20 smaller pathologically intact pieces. Monitoring is made using camera means introduced through yet another laparoscopic port, and the cut object pieces 17a,17b,....,17n may be collected in a receptacle introduced through yet another laparoscopic port, which receptacle is remove after the laparoscopic port has been removed. The whole surgical procedure will take about 10 min. allowing the surgeon to treat more patients than hitherto.

The bipolar electrosurgical instrument is, as mentioned above, particular suited for LASH, which is a partial hysterectomy that preserves the cervix, and the ovaries, removing only the diseased uterus. The ligaments and blood vessels are detached from the uterus and cauterized using an electrosurgical instrument. Next the uterus corpus is transected from the cervix at the level of the internal os. Instead of morcellating and removing the morcellated uterus with suction the resected uterus can according to the present invention be further sectioned and removed as intact pieces of a size allowed by the diameters of the laparoscopic ports. The risk of accidental injuring surrounding tissue e.g the rectum, bladder, and pelvic sidewall is minimal. If a monopolar electrosurgical instrument is used only one cutting action could have been made and a resected uterus must be removed either vaginally or by open surgery of sucked out after morcellation. Since LASH requires more cuttings to be completed monopolar electrosurgical cutting is not a safe option.

Although a preferred use of the instrument according to the present invention relates to female surgery, it is obvious that removal of other kinds of difficult accessible objects that are to be preserved as intact as possible is within the scope of the present invention. Such kinds of object could e.g. be submersed objects, plant objects, remote objects reachable via e.g. a robot, infectious plant and animal, etc.

## Claims

1. A bipolar electrosurgical instrument (1) being connectable to an electrosurgical generator (2), said bipolar electrosurgical instrument (1) comprising
- a hollow shaft (3) sized to fit through a laparoscopic port and having a proximal end (4) and a distal end (5),
- a loop electrode (8) constituted by an electrically conductive resilient looped wire provided with a first means (9) for reciprocating the loop electrode (8) between a retracted position inside the hollow shaft (3) and a second position in which at least a part of the loop electrode (8) is emerged from the distal end (5) of the hollow shaft (3), and
- at least two return electrodes (11a, 11b, 11c,11d) provided with second means (13a,13b,13c,13c,14) for reciprocating the at least two return electrodes (11a,11b,11c,11d) between a retracted position inside the hollow shaft (3) and a second position in which at least a part of the return electrodes (11a,11b,11c,11d) are emerged from the distal end (5) of the hollow shaft (3),
wherein, in use,
(a) the looped wire of the loop electrode can be arranged around a tissue object when the loop electrode is in the second position,
(b) and can then be constricted by retracting the loop electrode towards the retracted position, whereby the return electrodes obtain direct contact with the tissue object,
**characterised in that**
- the bipolar electrosurgical instrument (1) further comprises a third means (C) for measuring the impedance, resistance or capacity in the tissue object between the at least two return electrodes (11a,11b,11c,11d) when the return electrodes are in direct contact with the tissue object and
- a fourth means (B) for applying power to the loop electrode when said value of the impedance, resistance or capacity is below a predetermined value and/or in a predetermined period of time.

2. A bipolar electrosurgical instrument (1) according to claim 1, **characterised in that** the return electrodes (11a,11b,11c,11d) are arranged coaxilly to the loop electrode (8), at least when the loop electrode (8) is emerged from the proximal end (5) of the hollow shaft (3).

3. A bipolar electrosurgical instrument (1) according to any of the claims 1 or 2, **characterised in that** at least a part (8a,8b',8b") of the loop of the loop electrode (8) is arranged to be emerged between the emerged return electrodes (11a,11b,11c,11d).

4. A bipolar electrosurgical instrument (1) according to any of the claims 1,2 or 3, **characterised in that** the bipolar electrosurgical instrument (1) comprises at least four return electrodes (11a,11b,11c,11d).

5. A bipolar electrosurgical instrument (1) according to any of the preceding claims 1 - 4, **characterised in that** the return electrodes (11a,11b,11c,11d) are constituted by flat plates or blades 12a,12b,12c,12d, preferably of stainless steel.

6. A bipolar electrosurgical instrument (1) according to any of the preceding claims 1 - 5, the instrument further comprising the electrosurgical generator, the instrument being further **characterised in that** the fourth means (B) for applying power to the loop electrode (3) and the third means (C) for measuring any of the impedance, the resistance or capacity are integrated in the electrosurgical generator (2).

7. A bipolar electrosurgical instrument (1) according to any of the preceding claims 1 - 6, **characterised in that** the hollow shaft (3) comprises an outer tubular casing (6) having a longitudinal slide groove (13a, 13b, 13c, 13d) for reciprocating the return electrodes (11a,11b,11c,11d), and an inner tubular casing (7) accommodating the loop electrode (8) which inner tubular casing (7) is insulated from the outer tubular casing (6).

## Patentansprüche

1. Bipolares elektrochirurgisches Instrument (1), das an einen elektrochirurgischen Generator (2) anschließbar ist, wobei das bipolare elektrochirurgische Instrument (1) Folgendes umfasst:
- einen hohlen Schaft (3), der dimensioniert ist, um durch einen laparoskopischen Zugang zu passen, und der ein proximales Ende (4) und ein distales Ende (5) aufweist,
- eine Schlingenelektrode (8), die aus einem elektrisch leitfähigen, federnden Schlingendraht besteht, der mit einem ersten Mittel (9) versehen ist, um die Schlingenelektrode (8) zwischen einer eingezogenen Position im Innern des hohlen Schafts (3) und einer zweiten Position, in der mindestens ein Teil der Schlingenelektrode (8) aus dem distalen Ende (5) des hohlen Schafts (3) hervortritt, hin und her zu bewegen, und
- mindestens zwei Rücklaufelektroden (11a, 11b, 11c, 11d), die mit zweiten Mitteln (13a, 13b, 13c, 13d, 14) versehen sind, um die mindestens zwei Rücklaufelektroden (11a, 11b, 11c, 11d) zwischen einer eingezogenen Position im Innern des hohlen Schafts (3) und einer zweiten Position, in der mindestens ein Teil der Rücklaufelektroden (11a, 11b, 11c, 11d) aus dem distalen Ende (5) des hohlen Schafts (3) hervortritt, hin und her zu bewegen,
wobei im Gebrauch
(a) der Schlingendraht der Schlingenelektrode um ein Gewebeobjekt herum angeordnet werden kann, wenn sich die Schlingenelektrode in der zweiten Position befindet,
(b) und dann zusammengezogen werden kann, indem die Schlingenelektrode in Richtung auf die eingezogene Position eingezogen wird, wodurch die Rücklaufelektroden einen direkten Kontakt mit dem Gewebeobjekt erzielen können,
**dadurch gekennzeichnet, dass**
- das bipolare elektrochirurgische Instrument (1) ferner ein drittes Mittel (C) umfasst, um die Impedanz, den Widerstand oder die Kapazität in dem Gewebeobjekt zwischen den mindestens zwei Rücklaufelektroden (11a, 11b, 11c, 11d) zu messen, wenn sich die Rücklaufelektroden in direktem Kontakt mit dem Gewebeobjekt befinden, und
- ein viertes Mittel (B) umfasst, um Energie an die Schlingenelektrode anzulegen, wenn der Wert der Impedanz, des Widerstandes oder der Kapazität unter einem vorherbestimmten Wert und/oder in einem vorherbestimmten Zeitraum liegt.

2. Bipolares elektrochirurgisches Instrument (1) nach Anspruch 1, **dadurch gekennzeichnet, dass** die Rücklaufelektroden (11a, 11b, 11c, 11d) koaxial zur Schlingenelektrode (8) angeordnet sind, mindestens wenn die Schlingenelektrode (8) aus dem proximalen Ende (5) des hohlen Schafts (3) heraustritt.

3. Bipolares elektrochirurgisches Instrument (1) nach einem der Ansprüche 1 oder 2, **dadurch gekennzeichnet, dass** mindestens ein Teil (8a, 8b', 8b") der Schlinge der Schlingenelektrode (8) angeordnet ist, um zwischen den herausgetretenen Rücklaufelektroden (11a, 11b, 11c, 11d) herauszutreten.

4. Bipolares elektrochirurgisches Instrument (1) nach einem der Ansprüche 1, 2 oder 3, **dadurch gekennzeichnet, dass** das bipolare elektrochirurgische Instrument (1) mindestens vier Rücklaufelektroden (11a, 11b, 11c, 11d) umfasst.

5. Bipolares elektrochirurgisches Instrument (1) nach einem der vorhergehenden Ansprüche 1 bis 4, **dadurch gekennzeichnet, dass** die Rücklaufelektroden (11a, 11b, 11c, 11d) aus flachen Plättchen oder Lamellen (12a, 12b, 12c, 12d) bevorzugt aus Edelstahl bestehen.

6. Bipolares elektrochirurgisches Instrument (1) nach einem der vorhergehenden Ansprüche 1 bis 5, wobei das Instrument ferner den elektrochirurgischen Generator umfasst, wobei das Instrument ferner **dadurch gekennzeichnet ist, dass** das vierte Mittel (B) zum Anlegen von Energie an die Schlingenelektrode (3) und das dritte Mittel (C) zum Messen der Impedanz, des Widerstandes oder der Kapazität, in den elektrochirurgischen Generator (2) integriert sind.

7. Bipolares elektrochirurgisches Instrument (1) nach einem der vorhergehenden Ansprüche 1 bis 6, **dadurch gekennzeichnet, dass** der hohle Schaft (3) ein äußeres röhrenförmiges Gehäuse (6) umfasst, das eine längsgerichtete Gleitrille (13a, 13b, 13c, 13d) umfasst, um die Rücklaufelektroden (11a, 11b, 11c, 11d) hin und her zu bewegen, und ein inneres röhrenförmiges Gehäuse (7) umfasst, das die Schlingenelektrode (8) aufnimmt, wobei das innere röhrenförmige Gehäuse (7) gegenüber dem äußeren röhrenförmigen Gehäuse (6) isoliert ist.

## Revendications

1. Instrument électrochirurgical bipolaire (1) branchable à un générateur électro-chirurgical (2), ledit instrument électrochirurgical bipolaire (1) comprenant :
- un arbre creux (3) dont la taille a été choisie pour s'ajuster à travers un port laparoscopique et doté d'une extrémité proximale (4) et d'une extrémité distale (5),
- une électrode en forme de boucle (8) constituée par un fil élastique en boucle qui conduit le courant électrique fourni avec un premier moyen (9) pour donner un mouvement alternatif à l'électrode en forme de boucle (8) entre une position rétractée dans l'arbre creux (3) et une seconde position dans laquelle au moins une partie de l'électrode en forme de boucle (8) émerge de l'extrémité distale (5) de l'arbre creux (3), et
- au moins deux électrodes de retour (11a, 11b, 11c, 11d) fournies avec un second procéder (13a, 13b, 13c, 13c,14) pour donner un mouvement alternatif aux au moins deux électrodes de retour (11a,11b,11c,11d) entre une position rétractée dans l'arbre creux (3) et une seconde position dans laquelle au moins une partie des électrodes de retour (11a, 11b, 11c, 11d) émerge de l'extrémité distale (5) de l'arbre creux (3), où, pendant le fonctionnement,
(a) le fil en boucle de l'électrode en forme de boucle peut être disposé autour d'un objet en tissu quand l'électrode en forme de boucle est dans la seconde position,
(b) et peut alors être restreint en rétractant l'électrode en forme de boucle vers la position rétractée, situation dans laquelle les électrodes de retour se trouvent en contact direct avec l'objet en tissu,
**caractérisé en ce que**
- l'instrument électrochirurgical bipolaire (1) comprend en outre un troisième moyen (C) pour mesurer l'impédance, la résistance ou la capacité de l'objet en tissu entre les au moins deux électrodes de retour (11a, 11b, 11c, 11d) lorsque ces électrodes de retour sont en contact direct avec l'objet en tissu et
- un quatrième moyen (B) pour appliquer de l'énergie à l'électrode en forme de boucle lorsque ladite valeur d'impédance, de résistance ou de capacité est inférieure à une valeur prédéterminée et / ou pendant une période de temps prédéterminée.

2. Instrument électrochirurgical bipolaire (1) selon la revendication 1, **caractérisé en ce que** les électrodes de retour (11a,11b,11c,11d) sont disposées de manière coaxiale par rapport à l'électrode en forme de boucle (8), au moins lorsque l'électrode en forme de boucle (8) émerge de l'extrémité proximale (5) de l'arbre creux (3).

3. Instrument électrochirurgical bipolaire (1) selon l'une des revendications 1 ou 2, **caractérisé en ce qu'**au moins une partie (8a,8b',8b") de la boucle de l'électrode en forme de boucle (8) est disposée pour émerger entre les électrodes de retour émergées (11a,11b,11c,11d).

4. Instrument électrochirurgical bipolaire (1) selon l'une des revendications 1, 2 ou 3, **caractérisé en ce que** l'instrument électrochirurgical bipolaire (1) comprend au moins quatre électrodes de retour (11a,11b,11c,11d).

5. Instrument électrochirurgical bipolaire (1) selon l'une des précédentes revendications 1 à 4 , **caractérisé en ce que** les électrodes de retour (11a,11b,11c,11d) sont constituées de plaques ou lames plates 12a,12b,12c,12d, de préférence en acier inoxydable.

6. Instrument électrochirurgical bipolaire (1) selon l'une des précédentes revendications 1 à 5, l'instrument comprenant en outre le générateur électrochirurgical, l'instrument étant en outre **caractérisé en ce que** le quatrième moyen (B) d'application de l'énergie à l'électrode en forme de boucle (3) et le troisième moyen (C) pour mesurer l'une quelconque des valeurs suivantes, l'impédance, la résistance ou la capacité, sont intégrés dans le générateur électrochirurgical (2).

7. Instrument électrochirurgical bipolaire (1) selon l'une des précédentes revendications 1 à 6 , **caractérisé en ce que** l'arbre creux (3) comprend une enveloppe tubulaire externe (6) ayant une rainure de glissement longitudinal (13a,13b,13c,13d) pour conférer un mouvement alternatif aux électrodes de retour (11a,11b,11c,11d), et une enveloppe tubulaire interne (7) hébergeant l'électrode en forme de boucle (8), laquelle enveloppe tubulaire interne(7) étant isolée de l'enveloppe tubulaire externe (6).
